# EUROPEAN PATENT SPECIFICATION

(11) **EP 3 968 906 B1**
(45) Date of publication and mention of the grant of the patent: **10.07.2024**
(21) Application number: 19928547.9
(22) Date of filing: 22.10.2019
(51) Int. Cl.: A61F 2/50, A61L 15/26

(54) **IMPROVED SKIN SUBSTITUTE ADHERENCE, STRETCHABILITY, AND COMPLIANCE FOR SKIN SUBSTITUTES**
VERBESSERTE HAUTERSATZANHAFTUNG, DEHNBARKEIT UND NACHGIEBIGKEIT FÜR HAUTERSATZ
AMÉLIORATION DE L'ADHÉRENCE, DE L'APTITUDE À L'ÉTIRAGE ET DE LA COMPLIANCE DE SUBSTITUT CUTANÉ POUR SUBSTITUTS CUTANÉS

(30) Priority: 13.05.2019 US 201916410379
(43) Date of publication of application: 23.03.2022
(73) Proprietor: Woodroof, E. Aubrey, Carlsbad, CA 92009 (US)
(72) Inventor: Woodroof, E. Aubrey, Carlsbad, CA 92009 (US)
(74) Representative: Marks & Clerk LLP
(86) International application number: PCT/US2019/057491
(87) International publication number: WO 2020/231463

(56) References cited:
- WO-A1-2005/039464
- WO-A1-2014/138309
- WO-A1-2014/147638
- US-A1- 2010 331 751
- US-A1- 2013 299 220
- US-A1- 2014 343 676
- US-A1- 2016 022 560
- US-A1- 2016 346 133
- US-A1- 2018 140 468

## Description

### RELATED APPLICATIONS

This application is a Continuation-in-Part of US Pat. Appl. 16/410,379, filed May 13, 2019 and a Continuation-In-Part of US Pat. Appl. 15/396,720, filed January 2, 2017.

### FIELD OF THE INVENTION

This invention relates to primary dressings and bandages for both acute and chronic wounds, particularly for burns.

### BACKGROUND OF THE INVENTION

Over the past four and one-half decades of clinical use of bilaminate temporary skin substitutes, Biobrane was found to be an ideal replacement for human skin (FHCA - frozen human cadaver allograft which still is the "Gold Standard"). The secondary adherence at 72 hours of Biobrane (18/3 denier knitted nylon filament) was 200 grams per sq. cm. To vertically remove from the wound which is more than FHCA by about 50 grams per sq. cm. of force, e.g. Woodroof E.A. Biobrane; A Biosynthetic Skin Prosthesis. In Wise D. Ed. Burn Wound Coverage.

It is desirable to have secondary adherence near identical to FHCA on excised full-thickness wounds. In the last decade AWBAT^{®} (advanced wound bioengineered alternative tissue) - 15/2 denier and PermeaDerm^{®} (15/1 denier) have attempted to achieve the objective of enough but not too much secondary adherence. We have found a way to increase the thickness of PermeaDerm^{®} using 15/1 denier virgin nylon filament. A thicker 3D (three-dimensional) 15/1 denier PermeaDerm^{®} knitted structure enables greater adherence as well as increased stretchability and compliance. In the early 1980's I used 15/1 denier nylon to make a less adherent Biobrane for protection of widely meshed autografts, clinicians called it "light" Biobrane. Regular Biobrane was composed of 15/3 denier nylon. Also in the early 1980's International Paper Company created a silicone membrane bonded to a plush nylon velour material as opposed to a knitted nylon structure called IP-758 which had no biological coating nor drainage holes. Lack of drainage was related to increased infection complication. IP-758 was too adherent and faded from the market quickly. US 2016/346133 A1 discloses a skin substitute comprised of a silicone layer backed up with a woven nylon fabric layer.

### SUMMARY OF THE INVENTION

Aspects of the present invention provide for a bilaminate cured silicon/nylon material, as set out in the appended set of claims.

The present invention is a new approach to providing a glove and bootie bandage that addresses burn injuries. The state-of-art glove bandages do not possess the new features of the present invention, directed to allowing a measured amount of porosity, including essentially zero porosity, and a lower cost to produce combined with shorter healing/rehabilitation times of the burned hand.

The goal was to create a more effective biosynthetic glove for hand burns management. An almost perfect anatomical fit for the hand reducing operating room time and general anesthesia. A hydrophilic/hygroscopic moist, but not wet, wound healing environment which enhances healing.

The present invention enables faster and better wound closure to fitting thumb and 3 fingers initially; then the clinician completes closure with staples using greater circumferential elongation of the PermeaDerm^{®} B (burn) material plus extra material on distil portion of hand. The PermeaDerm^{®} B material is a more sophisticated biological coating than competing materials such as Biobrane or AWBAT/AWBAT^{®} Plus.

Wound sites have variable amounts of exudate/transudate/plasma present, from dry to weepy. The clinician must cleanly debride the wound, close it and manage wound healing in a moist but not wet environment to achieve optimal results for both acute and chronic wounds. This is particularly difficult with complicated structures like hands and feet.

The present invention provides a dressing that possesses all the properties and attributes of an ideal skin substitute and, in addition, has "variable porosity" controlled by the clinician from zero porosity to what the wound requires. The present invention enables the clinician to move the fluid exuding from the wound through the primary dressing into an absorbent secondary dressing without disturbing the kinetics of healing or causing pain to the patient.

The present invention may have a single layer of biologicals comprised of a hypoallergenic BSE free USP Pharmaceutical grade gelatin, pure Aloe or Aloesin, pure Aloe and BSE free gelatin, or a mixture of pure Aloe, BSE free gelatin and ECM interact with the wound to stimulate the rate of healing while adherent to the wound. The bio-coat layer may be deposited directly on the nylon side of the "variable porosity" silicone/nylon surface and is stable for over three years at room temperature.

*In vitro,* the Aloe component has been demonstrated to cause a variety of cells to attach and proliferate; as well as increase synthesis of collagen and alpha smooth muscle actin. ECM may be added to the biologicals described above and is a mixture from human fibroblasts that is known to cause rapid cell proliferation and tissue growth. Previous wound dressings and skin substitutes, as taught in US Pat. 7,815,931 contain gelatin, a pure Aloe component, chondroitin 4 & 6 sulfate, and vitamin C & E. In contrast the current dressing will have two layers of biologicals applied in separate spraying operations as described above. The first coat will contact the wound after the second coat of hypoallergenic bovine spongiform encephalopathy (BSE) - free United States Pharmaceutical (USP) -grade gelatin interacts with fibrin in the wound to achieve early adherence. The second coat of biologicals stimulates the healing process during the interval where the dressing invention is in contact with the wound and is stable requiring 100 degree water for 30 minutes to remove from the "variable porosity" silicone/nylon surface.

Human wounds vary in the following ways depending on location: relatively flat, convex or concave surface; and vary in movement from minimal to a lot (diaphragm). Most difficult location to achieve adherence are convex wounds where there is movement (axilla, upper abdomen, etc.). Irregular depth wounds such as ulcers (DFU, VLU-diabetic foot ulcer and venous leg ulcer) require prompt closure requiring excellent compliance, stretchability or elongation.

If slow-healing ulcers (DFU, VLU, PU - pressure ulcer) are not closed promptly infection complications may often occur and potential loss of limb and compromise of life style is at risk. Clinical preference is current PDB (average thickness is 0.10922mm (0.0043") with STDEV of 0.00762mm (0.0003")) for protection of widely meshed autograft where maximization of healing (definitive closure) of the interstices is desired. Too much adherence (greater than 50 gm per sq. cm. at 5 hours or 0.12192mm (0.0048") thick) will slow interstices healing.

The PermeaDerm IFU (instructions For Use) describe immobilization of PermeaDerm for initial 24 hours to maximize early adherence. This is commonly done except for the neck and abdomen adjacent to diaphragm. Bolstering techniques are frequently used to hold the bio-coated surface in intimate contact with the debrided or excised hemostatic wound until adherence.

A thicker 3D structure (average thickness 0.2032mm (0.0080") with STDEV 0.02032mm (0.0008")) for PDG (PermeaDerm^{®} glove) provides additional advantages over normal thickness PDG with respect to greater circumferential elongation and fit in the web spaces giving the clinician greater room for error during the fitting process. Once properly fitted, PDG will enable rapid rehabilitation without pain to regain full range of motion and will minimize interference with the healing process yielding excellent aesthetic and functional results. PDB and PDC (PermeaDerm^{®} Chronic) would clinically benefit from both greater adherence and greater elongation/conformability.

There is a regulatory advantage (FDA approval) based on use of same material (15/1 denier virgin nylon filament) to increase thickness of 3D structure of PermeaDerm^{®} product as opposed to use of a different denier of virgin nylon filament (example 15/2, 15/3, 18/3 denier). Estimated thickness using SEM: 15/1 denier about 0.0069" and 18/3 denier about 0.3556mm (0.014"). 3D structure of 18/3 denier is about 103% (more than double) thicker than 15/1 denier.

### BRIEF DESCRIPTION OF THE FIGURES

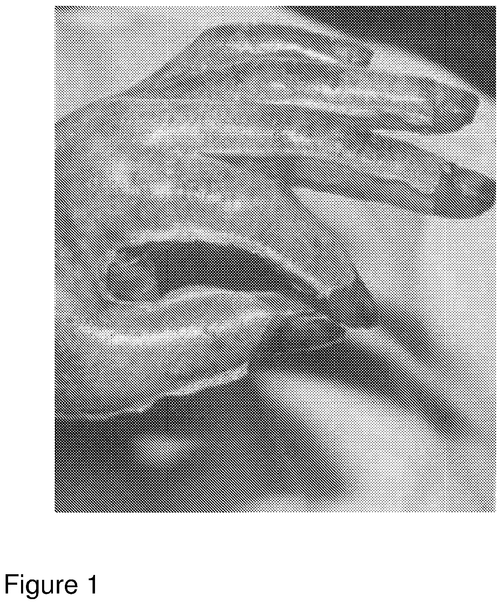

Figure 1 - The Biobrane^{®} Glove - fabricated using two sheets of planar Biobrane 1982.

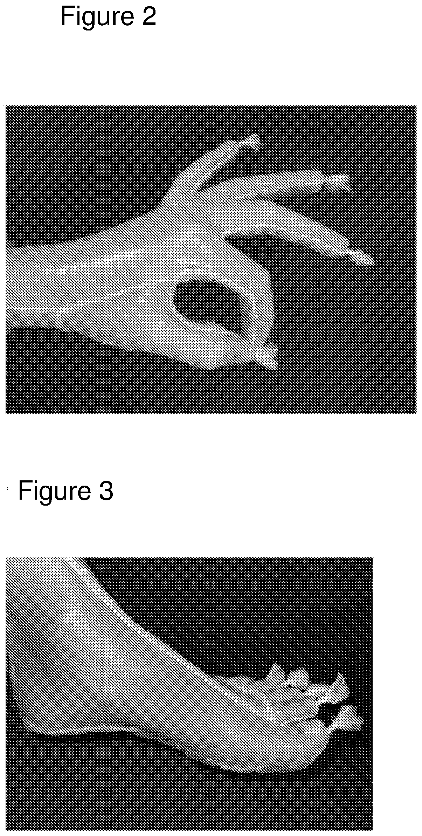

Figure 2 - AWBATO Glove, a precision anatomical fit Biosynthetic Glove with three-piece design in 2009. PermeaDerm^{®} Glove is more practical and effective.
Figure 3 - AWBAT^{®} Bootie, a precision anatomical fit with three-piece design 2009. PermeaDerm^{®} Bootie is more practical and effective.

### DETAILED DESCRIPTION

The present invention is as defined by the claims. The preferred embodiment is a glove made by gluing two pieces of PermeaDerm^{®} together with the biologically coated 3D Gen 3 nylon structure facing each other and the slitted silicone surface facing out, the 3D coated nylon surface contacts the wound surface inside the glove. For lower cost and efficiency, glue is preferred to seams sewn with thread.

The present invention is similar in composition to earlier skin substitutes in that they each have a thin silicone component and an underlying 3D thin knitted nylon component. The present invention differs from its ancestors in that it has "variable porosity" controlled by the clinician; the slit width in the thin silicone will be 37260911-1 essentially zero (with no stretch, in relaxed mode) to a higher porosity (proportional to the stretch applied).

In addition, the present invention differs from prior art in the composition of biological coatings applied to both components and how these coatings interact with the wound over time.

The preferred embodiment of the invention designed for burns, a regular pattern with slits on the silicone surface parallel to each other in the same row is presented. The slits made in the silicone are approximately. 3.175mm (0.125") long with a space of 12.7mm (0.50"), between the slits; off-set parallel rows of slits are 6.35mm (0.25") apart.

In this configuration the silicone/nylon membrane can be stretched in a direction perpendicular to the slit orientation and the slits will open. Porosity therefore increases proportionally to the amount of stretch applied. Obviously, there is a maximum amount of stretching of the preferred embodiment before the dressing fails. IFU (instructions for use) describe a maximum of 20% elongation, well below the breaking point.

The use of the present invention has a large benefit because it is stable on the wound and possesses biologicals that aid in the healing process.

The present invention will have a single layer of biologicals comprised of a hypoallergenic BSE free USP Pharmaceutical grade gelatin, pure Aloe or Aloesin, pure Aloe and BSE free gelatin, or a mixture of pure Aloe, BSE free gelatin and ECM interact with the wound to stimulate the rate of healing while adherent to the wound. The layer is deposited directly on the nylon side of the "variable porosity" silicone/nylon surface and is stable for at least three years at room temperature.

The biologicals are applied to the knitted nylon side of the present embodiment. Optionally, the biologicals can be applied to the silicone layer as well.

Hand burns represent a small wound area with major potential for disability. The management of hand burns is multidisciplinary, requiring the expertise of surgeons, nurses, and occupational therapists. In addition to surgical excision and autografting, several wound healing modalities are available for the care of hand burns including a bilaminate, biosynthetic glove.

Motivated and reliable patients with burns limited to the hands can be appropriately managed on an outpatient basis at a burn center, where the requisite expertise in wound care, excision and grafting, and occupational therapy are available. An increase in elongation and compliance (greater 3D structure) of PermeaDerm results in improvements of the following:
3D thickness of cured silicone nylon membrane: Preliminary thickness data of cured silicone/nylon membrane from the manufacturer, January 9, 2019 using micrometer:
Gen1 - Mean 0.10922mm (0.0043"), STDEV 0.00762mm (0.0003"), Min 0.1016mm (0.0040"), Max 0.12192mm (0.0048"). Gen3 - Mean 0.2032mm (0.0080"), STDEV 0.02032mm (0.0008"), Min 0.1778mm (0.0070"), Max 0.2413mm (0.0095"). 3D structure of Gen3 is about 86% thicker than Gen1. Conclusions on clinically effective adherence relative to thickness of a bilaminate silicone/nylon membrane:
O Less than 0.0040" - **Inadequate** adherence
O Current PDB and PDC - **Adequate** adherence - average thickness 0.10922mm (0.0043") with STDEV 0.00762mm (0.0003")
O PDB, PDC and PDG with Gen3 nylon - **Optimal** Adherence -average thickness 0.2032mm (0.0080") with STDEV of 0.02032mm (0.0008")
O **Excessive** adherence - thickness greater than 0.2413mm (0.0095")

**The** knitted nylon according to the invention **is the Gen3 PermeaDerm with average thickness of 0.2032mm (0.0080") and STDEV of 0.02032mm (0.0008") which results in Optimal adherence.**

### Before Slitting

| | **Gen1** | **Gen3** | **Gen3/Gen1** |
|---|---|---|---|
| **Average** | **0.10922mm (0.0043")** | **0.2032mm (0.0080")** | **1.86** |
| **STDEV** | **0.00762mm (0.0003")** | **0.02032mm (0.0008")** | **2.67** |
| **Max** | **0.11684mm (0.0046")** | **0.2413mm (0.0095")** | |
| **Min** | **0.1016mm (0.0040")** | **0.1778mm (0.0070")** | |

The glove and/or bootie made with an average of 0.2032mm (0.008 inch) thickness nylon mesh is according to the invention. The technology to create the invention is listed in the preferred embodiment of this invention, but other methods are possible and are within the contemplation of this patent.

## Claims

1. A bilaminate cured silicone/nylon material,
the silicone/nylon material comprised of two layers of material,
the first layer of material comprised of a silicone membrane, the second layer comprised of a woven fabric,
the two layers combined together with a combination means such that the two layers form a single structure, the preferred combination method is heating, the first layer possessing a plurality of slits in its surface, said slits made after the two layers are combined, said slits in a regular pattern, the regular pattern comprising a parallel vertical orientation,
said first layer and said second layer treated with a coating comprised of one or more medicinal or therapeutic substances,
said coating possessing an additional component of salinomycin, native high molecular weight collagen, and Mafenide Acetate,
the porosity of said skin substitute minimized to essentially zero porosity in the mode where no stretching tension is exerted on the skin substitute, the porosity of said skin substitute variable proportional to the amount of stretching tension and the direction in which said stretching tension is placed on the skin substitute,
said second layer of material with a range of thickness of
15/1 denier knitted nylon of an average of 0.2032mm (0.0080") with STDEV of 0.02032mm (0.0008").

2. A bilaminate cured silicone/nylon material as in Claim 1 where said coating is a single layer of biological substances comprised of:
a. a hypoallergenic BSE free USP Pharmaceutical grade gelatin,
b. pure Aloe orAloesin,
c. pure Aloe and BSE free gelatin, or a mixture of pure Aloe, BSE free gelatin and ECM; and
where said coating interacts with a wound to stimulate the rate of healing while adherent to the wound, the coating deposited directly on the woven fabric, said coating chemically stable for at least three years at room temperature.

## Patentansprüche

1. Zweischichtiges, gehärtetes Silikon/Nylon-Material,
wobei das Silikon/Nylon-Material aus zwei Materialschichten besteht,
wobei die erste Materialschicht aus einer Silikonmembran besteht,
wobei die zweite Schicht aus einem Gewebe besteht,
wobei die zwei Schichten mit einem Kombinationsmittel miteinander kombiniert sind, so dass die zwei Schichten eine einzige Struktur bilden, wobei das bevorzugte Kombinationsverfahren Erhitzen ist,
wobei die erste Schicht eine Vielzahl von Schlitzen in ihrer Oberfläche aufweist, die Schlitze nach dem Kombinieren der zwei Schichten hergestellt werden, wobei die Schlitze ein regelmäßiges Muster aufweisen, wobei das regelmäßige Muster eine parallele vertikale Ausrichtung umfasst,
wobei die erste Schicht und die zweite Schicht mit einer Beschichtung behandelt sind, die aus einer oder mehreren medizinischen oder therapeutischen Substanzen besteht,
wobei die Beschichtung eine zusätzliche Komponente aus Salinomycin, nativem hochmolekularem Collagen und Mafenidacetat aufweist,
wobei die Porosität des Hautersatzes in dem Modus, in dem keine Dehnungsspannung auf den Hautersatz ausgeübt wird, auf im Wesentlichen null Porosität minimiert ist, wobei die Porosität des Hautersatzes proportional zur Menge der Dehnungsspannung und der Richtung, in der die Dehnungsspannung auf den Hautersatz ausgeübt wird, variabel ist,
wobei die zweite Materialschicht einen Dickenbereich von 15/1 Denier gestricktem Nylon von durchschnittlich 0,2032 mm (0,0080") mit einer Standardabweichung von 0,02032 mm (0,0008") aufweist.

2. Zweischichtiges, gehärtetes Silikon-/Nylonmaterial nach Anspruch 1, wobei die Beschichtung eine einzige Schicht aus biologischen Substanzen ist, bestehend aus:
a. einer hypoallergene, BSE-freie Gelatine in pharmazeutischer USP-Qualität,
b. reiner Aloe oder Aloesin,
c. reiner Aloe und BSE-freie Gelatine oder einem Gemisch aus reiner Aloe, BSE-freier Gelatine und ECM; und
worin die Beschichtung mit einer Wunde in Wechselwirkung tritt, um die Heilungsrate zu stimulieren, während sie an der Wunde haftet, wobei die Beschichtung direkt auf dem Gewebe aufgebracht ist, wobei die Beschichtung bei Raumtemperatur mindestens drei Jahre lang chemisch stabil ist.

## Revendications

1. Matériau de silicone / nylon durci bistratifié,
le matériau de silicone / nylon étant constitué de deux couches de matériau,
la première couche de matériau étant constituée d'une membrane de silicone,
la deuxième couche étant constituée d'un tissu tissé,
les deux couches étant combinées ensemble avec un moyen de combinaison de sorte que les deux couches forment une structure unique, le procédé de combinaison préféré étant le chauffage,
la première couche possédant une pluralité d'encoches dans sa surface, lesdites encoches étant réalisées après que les deux couches sont combinées, lesdites encoches ayant un motif régulier, le motif régulier comprenant une orientation verticale parallèle,
ladite première couche et ladite deuxième couche étant traitées avec un revêtement constitué d'une ou plusieurs substances médicinales ou thérapeutiques,
ledit revêtement contenant un constituant supplémentaire de salinomycine, de collagène à haut poids moléculaire natif, et d'acétate de mafénide,
la porosité dudit substitut cutané étant réduite à une porosité sensiblement nulle dans un mode où aucune tension d'étirement n'est exercée sur le substitut de peau, la porosité dudit substitut cutané étant variable proportionnellement à la quantité de tension d'étirement et la direction dans laquelle la tension d'étirement est appliquée au substitut cutané,
ladite deuxième couche de matériau ayant une gamme d'épaisseur de nylon tricoté de 15/1 deniers d'une moyenne de 0,2032 mm (0,0080") avec un écart-type de 0,02032 mm (0,0008").

2. Matériau de silicone / nylon durci bistratifié selon la revendication 1, dans lequel ledit revêtement est une couche unique de substances biologiques constituée :
a. d'une gélatine de qualité pharmaceutique USP hypoallergénique exempte d'ESB,
b. d'aloès pur ou d'aloésine pure,
c. d'aloès pur et de gélatine exempte d'ESB, ou d'un mélange d'aloès pur, de gélatine exempte d'ESB et d'ECM, et
où ledit revêtement interagit avec une blessure pour stimuler la vitesse de cicatrisation lorsqu'il est adhérent à la blessure, le revêtement étant déposé directement sur le tissu tissé, ledit revêtement étant chimiquement stable pendant au moins trois ans à température ambiante.
